(19) Europäisches Patentamt

European Patent Office

Office européen des brevets

(11) EP 1 209 612 A2

(12) **EUROPEAN PATENT APPLICATION**

(43) Date of publication:
29.05.2002 Bulletin 2002/22

(51) Int Cl.$^7$: **G06F 19/00**

(21) Application number: **01309785.2**

(22) Date of filing: **21.11.2001**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **21.11.2000 US 721042**

(71) Applicant: **Affymetrix, Inc.**
**Santa Clara, CA 95051 (US)**

(72) Inventor: **Hunt, Nathaniel**
**Oakland, California 94610 (US)**

(74) Representative: **Chapman, Paul William et al**
**Kilburn & Strode,**
**20 Red Lion Street**
**London WC1R 4PJ (GB)**

(54) **Methods and computer software products for predicting nucleic acid hybridization affinity**

(57)    Methods and computer software products are provided for predicting nucleic acid hybridization affinity. In one embodiment, hybridization intensity (I) is determined using the equation:

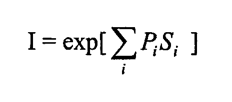

where Pi is the value of the ith parameter and Si is a value derived from the sequence of the probe. The methods and software products of the invention may be used for enhancing mutation detection, probe selection and probe array manufacturing quality control.

$$I = \exp\left[\sum_i P_i S_i\right]$$

**Figure 3a**

**Description**

**U.S. GOVERNMENT INTERESTS**

This invention was made with government support under Grant number _____ from the National Institutes of Health. Accordingly, the U.S. Government retains certain rights in the invention.

**BACKGROUND OF THE INVENTION**

The present invention relates to methods for analyzing DNA microarray data and for designing DNA microarrays.

U.S. Patent No. 5,424,186 describes a pioneering technique for, among other things, forming and using high density arrays of molecules such as oligonucleotide, RNA, peptides, polysaccharides, and other materials. This patent is hereby incorporated by reference for all purposes.

Arrays of oligonucleotides or peptides, for example, are formed on the surface by sequentially removing a photoremovable group from a surface, coupling a monomer to the exposed region of the surface, and repeating the process. These techniques have been used to form extremely dense arrays of oligonucleotides, peptides, and other materials. The synthesis technology associated with this invention has come to be known as "VLSIPS™ " or "Very Large Scale Immobilized Polymer Synthesis" technology.

Additional techniques for forming and using such arrays are described in U.S. Patent Nos. 5,384,261, and 6,040,193 which are also incorporated by reference for all purposes. Such techniques include systems for mechanically protecting portions of a substrate (or chip), and selectively deprotecting/coupling materials to the substrate. Still further techniques for array synthesis are provided in U.S. Application No. 08/327,512, also incorporated herein by reference for all purposes.

Nucleic acid probe arrays have found wide applications in gene expression monitoring, genotyping and mutation detection. For example, massive parallel gene expression monitoring methods using nucleic acid array technology have been developed to monitor the expression of a large number of genes (e.g., U.S. Patent Numbers 5,871,928, 5,800,992 and 6,040,138; de Saizieu *et al.,* 1998, Bacteria Transcript Imaging by Hybridization of total RNA to Oligonucleotide Arrays, NATURE BIOTECHNOLOGY, 16:45-48; Wodicka *et al.,* 1997, Genome-wide Expression Monitoring in Saccharomyces cerevisiae, NATURE BIOTECHNOLOGY 15:1359-1367; Lockhart *et al.,* 1996, Expression Monitoring by Hybridization to High Density Oligonucleotide Arrays. NATURE BIOTECHNOLOGY 14:1675-1680; Lander, 1999, Array of Hope, NATURE-GENETTCS, 21(suppl.), at 3, all incorporated herein by reference for all purposes). Hybridization-based methodologies for high throughput mutational analysis using high-density oligonucleotide arrays (DNA chips) have been developed, see Hacia *et al.,* 1996, Detection of heterozygous mutations in BRCA1 using high density oligonucleotide arrays and two-color fluorescence analysis. Nat. Genet. 14:441-447, Hacia *et al.*, New approaches to BRCA1 mutation detection, Breast Disease 10:45-59 and Ramsey 1998, DNA chips: State-of-Art, Nat Biotechnol. 16:40-44, all incorporated herein by reference for all purposes). Oligonucleotide arrays have been used to screen for sequence variations in, for example, the *CFTR* gene (U.S. Patent Number 6,027,880, Cronin et al., 1996, Cystic fibrosis mutation detection by hybridization to light-generated DNA probe arrays. Hum. Mut. 7:244-255, both incorporated by reference), the human immunodeficiency virus (HIV-1) reverse transcriptase and protease genes (U. S. Patent Number 5,862,242 and Kozal et al., 1996, Extensive polymorphisms observed in HIV-1 clade B protease gene using high density oligonucleotide arrays. Nature Med. 1:735-759, both incorporated herein by reference for all purposes), the mitochondrial genome (Chee et al., 1996, Accessing genetic information with high density DNA arrays. Science 274:610-614) and the BRCA1 gene (U.S. Patent Number 6,013,449, incorporated herein by reference for all purposes)

**SUMMARY OF THE INVENTION**

This invention provides methods and software products for predicting hybridization affinity of a nucleic acid probe against its target. In preferred embodiments, the probe is immobilized on a solid substrate. In most preferred embodiments, the probe is part of a nucleic acid probe array, such as synthetic oligonucleotides on a glass substrate, on optical fibers or beads. The methods and software have extensive applications in enhancing mutation detection, selecting probes for probe arrays, and enhancing quality control of probe array manufacturing process.

In one aspect of the invention, computer implemented methods for predicting hybridization affinity of a probe against its target is provided. The methods include a step of calculating hybridization intensity (I) using the equation:

$$I = \exp\left[\sum_i P_i S_i\right]$$

wherein Pi is the value of the ith parameter and Si is a value derived from the sequence of the probe. In some embodiments, Pi is the free energy of a base in a given position of the sequence relative to a reference base selected from the group consisting of A, C, G and T. Si may be a functional of said sequence. The Pi may be determined empirically, for example, using least square fit of a training data set. Pi values may be determined using Cholesky decomposition. In some embodiments, Si for a given nucleotide type is replaced with a smooth function of probe base number. The smooth function may be a polynomial function. In some other embodiments, the Si for a given nucleotide type is replaced with a smooth function of the difference of a base to the central position. In some additional embodiments, the equation includes at least one parameter describing probe-probe hybridization and/or at least one parameter describing hairpin formation. In some other preferred embodiment, the equation includes parameters describing nearest neighbor interactions. In such embodiments, Pi values may be determined using singular value decomposition.

In another aspect of the invention, computer implemented methods for selecting probes for gene expression monitoring is provided. The methods include a step of predicting hybridization intensities of a plurality of candidate probes and their corresponding control probes; and a step of selecting the candidate probe that has the highest intensity difference over its corresponding control probe. Alternatively, the intensity difference may be directly predicted.

The hybridization intensity (I) is determined using the equation:

$$I = \exp\left[\sum_i P_i S_i\right]$$

wherein Pi is the value of the ith parameter and Si is a value derived from the sequence of the probe. The Pi is the free energy of a base in a given position of said sequence relative to a reference base selected from the group consisting of A, C, G and T. Si is a functional of the sequence of the probe. Pi is determined empirically, for example, using least square fit of a training data set. Pi values may be determined using Cholesky decomposition. In some embodiments, Si for a given nucleotide type is replaced with a smooth function of probe base number. Exemplary smooth functions include polynomial functions. In some other embodiments, Si for a given nucleotide type is replaced with a smooth function of the difference of a base to the central position. In some additional embodiments, the equation includes at least one parameter describing probe-probe hybridization, at least one parameter describing hairpin formation, and/or parameters describing nearest neighbor interactions. If the equation includes parameters describing nearest neighbor interactions, the Pi values may be determined using singular value decomposition.

In another aspect of the invention, computer software products for predicting hybridization affinity of a probe against its target are provided. The software may be written in any of various suitable computer programming languages such as C, C++, Fortran, Basic, and Java. Software components such as Java Beans, enterprise Java Beans, etc., may be employed as a part of the architecture of the software products of the invention.

The software products include computer program code for calculating hybridization intensity (I) using the equation:

$$I = \exp\left[\sum_i P_i S_i\right]$$

wherein Pi is the value of the ith parameter and Si is a value derived from the sequence of the probe and a computer readable media for storing the computer program code. One of skill in the art would appreciate that code for displaying result of the calculation may also be included. Pi is the free energy of a base in a given position of the sequence relative to a reference base selected from the group consisting of A, C, G and T. Si is a functional of the sequence. Pi values are determined empirically, for example, by least square fit using a training data set, using Cholesky decomposition. In some embodiments, Si for a given nucleotide type is replaced with a smooth function of probe base number, such as a polynomial function. In some other embodiments, Si for a given nucleotide type is replaced with a smooth function of the difference of a base to the central position.

In some embodiments, the equation includes at least one parameter describing probe-probe hybridization, at least one parameter describing hairpin formation and/or parameters describing nearest neighbor interactions. If the equation include parameters describing nearest neighbor interaction, Pi values are determined using singular value decomposition.

In yet another aspect of the invention, computer software products for selecting probes for gene expression monitoring are provided. The software product include computer program code for predicting hybridization intensities of a plurality of candidate probes and their corresponding control probes; computer program code for selecting the candidate probe that has the highest intensity difference over its corresponding control probe; and a computer readable media for storing the codes. The computer code for predicting hybridization intensity (I) including code for calculating using the equation:

$$I = \exp\left[\sum_i P_i S_i \ \right]$$

wherein Pi is the value of the ith parameter and Si is a value derived from the sequence of said probe. In some embodiments, Pi is the free energy of a base in a given position of said sequence relative to a reference base selected from the group consisting of A, C, G and T. Si is a functional of the sequence of the probe. Pi values are determined empirically by, for example, least square fit of a training data set using Cholesky decomposition. Si for a given nucleotide type is replaced with a smooth function of probe base number. The smooth function may be a polynomial function. In other embodiments, Si for a given nucleotide type is replaced with a smooth function of the difference of a base to the central position. In some embodiments, the equation may include at least one parameter describing probe-probe hybridization, at least one parameter describing hairpin formation and/or parameters describing nearest neighbor interactions. If the equation includes parameters describing nearest neighbor interactions, Pi values may be determined using singular value decomposition.

## BRIEF DESCRIPTION OF THE DRAWINGS

The accompanying drawings, which are incorporated in and form a part of this specification, illustrate embodiments of the invention and, together with the description, serve to explain the principles of the invention:

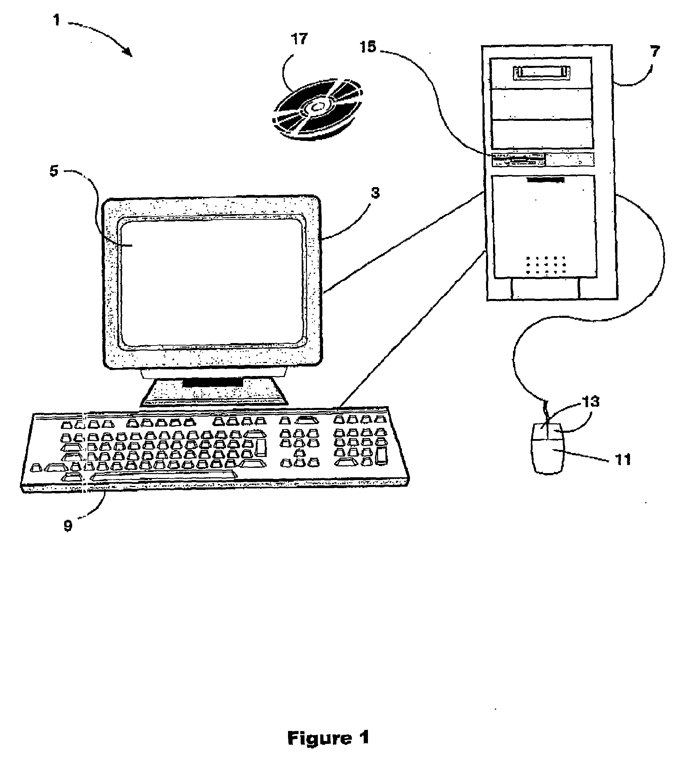

Figure 1 illustrates an example of a computer system that may be utilized to execute the software of an embodiment of the invention.

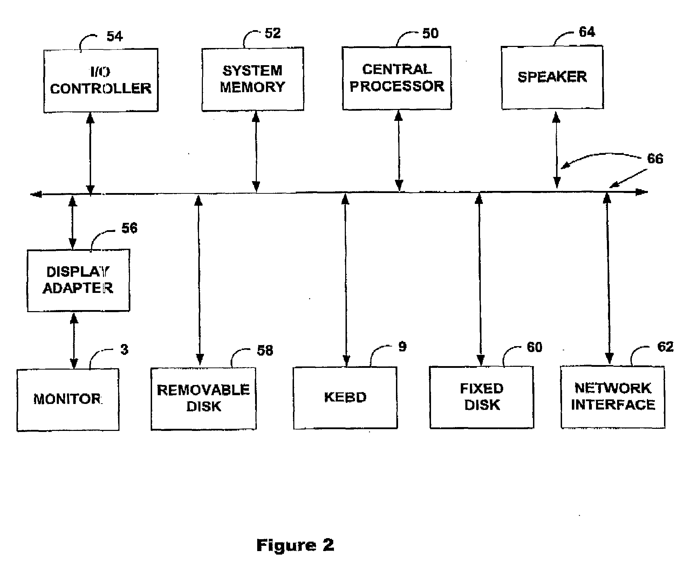

Figure 2 illustrates a system block diagram of the computer system of Fig. 1.

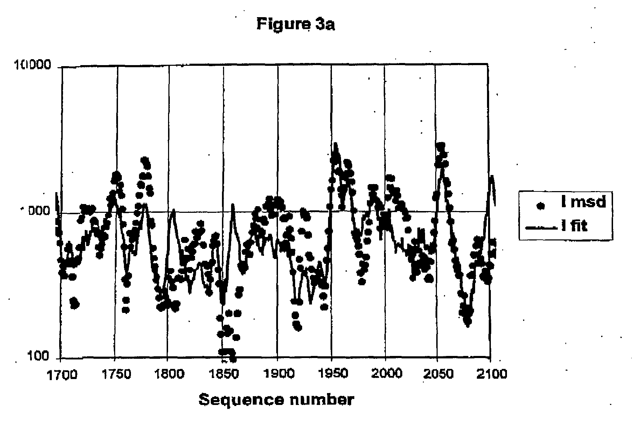

Figure 3a shows measured and fitted intensities for 400 bases (out of a total of 5.6 kb) of the BRCA1 sequence. 97 parameters are used in the fit -- 75 base-type and position parameters (a parameter for each non-T base at each of the 25 probe positions) and one normalization parameter for each of the 22 exons. The separate normalization parameters for each exon allow for the fact that different exons may be present at different concentrations. The root mean square (r.m.s.) deviation of the (natural) logs of $I_{fit}$ and $I_{msd}$ over the entire sequence is 0.8565.

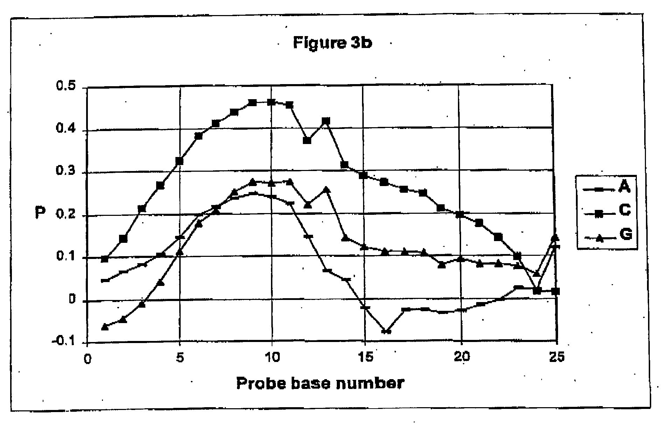

Figure 3b shows the 75 base- and position-specific parameters used to calculate the fitted intensities in Figure 3a.

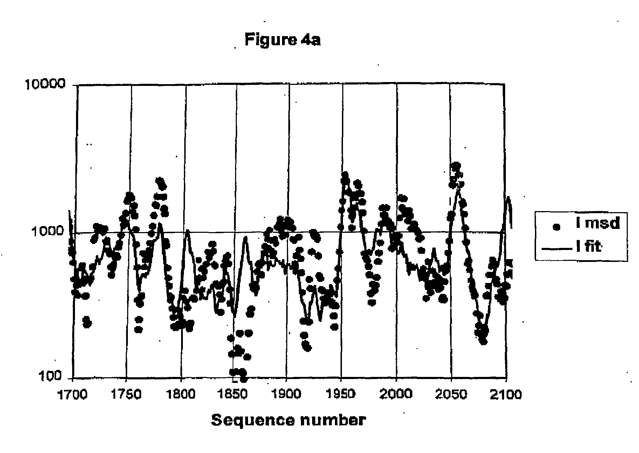

Figure 4a shows measured and fitted intensities for a portion of the BRCA1 sequence. 37 parameters are used in the fit -- 15 specify the three fourth-degree polynomials describing the base-and position-dependence of the hybridization, and the remaining 22 are normalization parameters for each of the 22 exons. The r.m.s. deviation of the logs of $I_{fit}$ and $I_{msd}$ over the entire sequence is 0.8712.

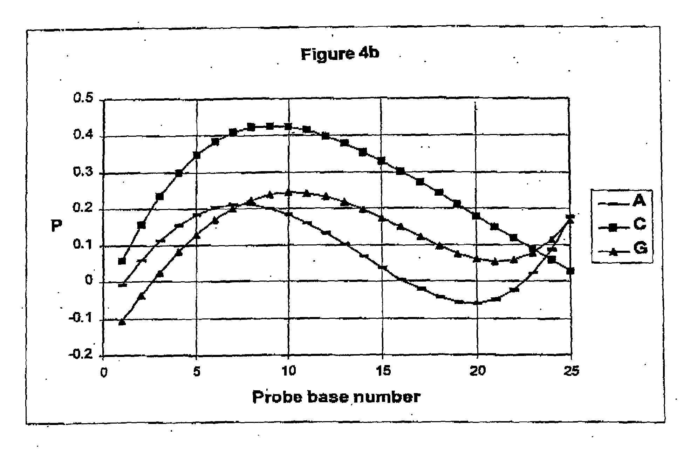

Figure 4b shows the base- and position-dependent hybridization free energies derived from the fitting parameters used in Figure 4a. Each curve is a fourth degree polynomial specified by five of the fitting parameters. These curves are lower information content versions of those shown in Figure 3b.

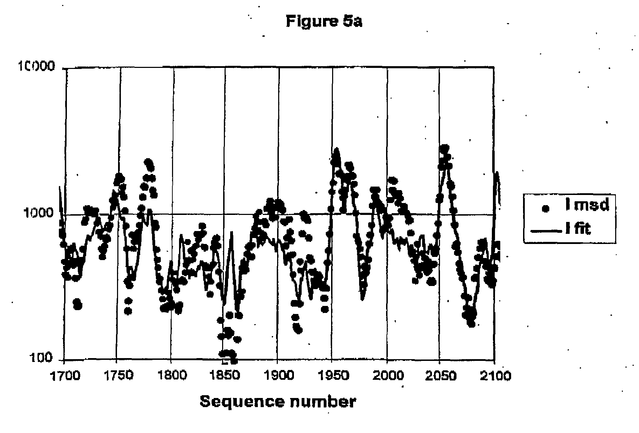

Figure 5a shows measured and fitted intensities for a portion of the BRCA1 sequence. 39 parameters are used in the fit -- 37 of them are as in Figure 4, and two additional ones describe probe-probe interactions and hairpin formation. The r.m.s. deviation of the logs of $I_{fit}$ and $I_{msd}$ over the entire sequence is 0.8274.

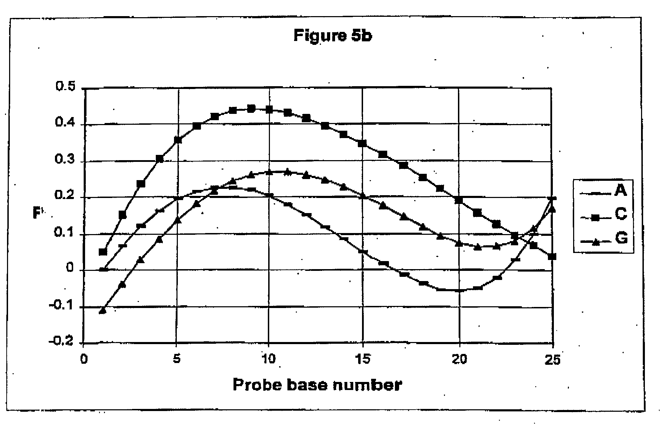

Figure 5b shows the base- and position-dependent hybridization free energies derived from the fitting parameters used in Figure 5a.

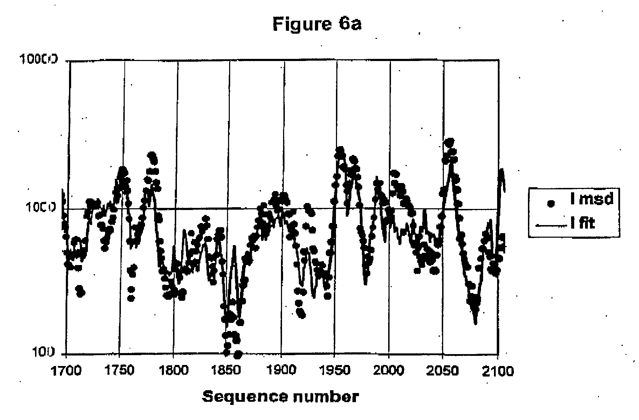

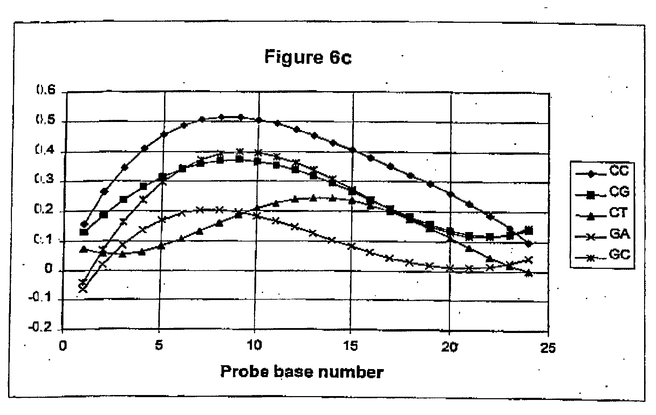

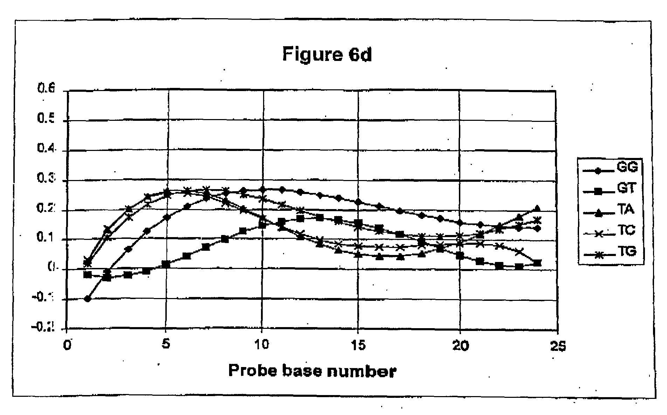

Figure 6a shows measured and fitted intensities for a portion of the BRCA1 sequence. 99 parameters are used in the fit -- 75 specify the 15 fourth-degree polynomials describing the base- and position-dependence of each non-reference nearest-neighbor term, two describe probe-probe interactions and hairpin formation, and 22 are normalization parameters for each of the 22 exons. The r.m.s. deviation of the logs of $I_{fit}$ and $I_{msd}$ over the entire sequence is 0.7436.

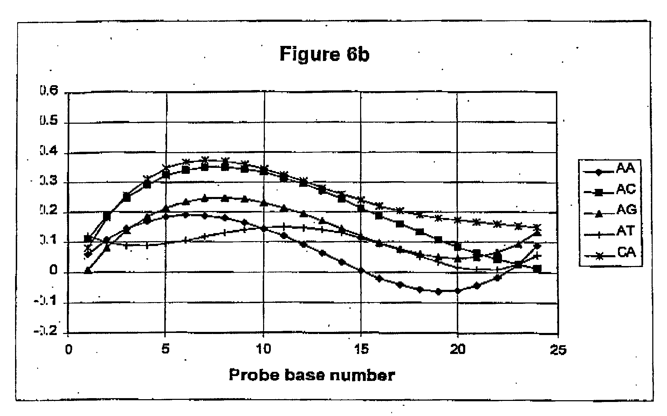

Figures 6b-d show the base- and position-dependent nearest-neighbor hybridization free energies derived from the fitting parameters used in Figure 6a.

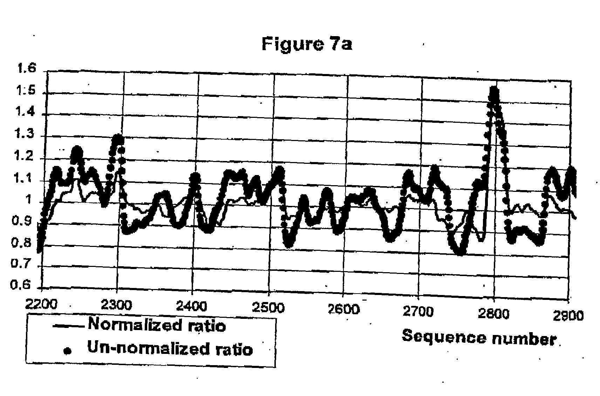

Figure 7a shows un-normalized and sequence-based normalized one-color ratios for a portion of the BRCA1 sequence. The sequence-based normalization used the same 37 parameter types used in Figure 5. Ratios above 1.3 are called as mutations. The sequence-based normalization eliminates a false positive just below sequence number 2300 while leaving the signal of the true mutation at position 2798 undiminished.

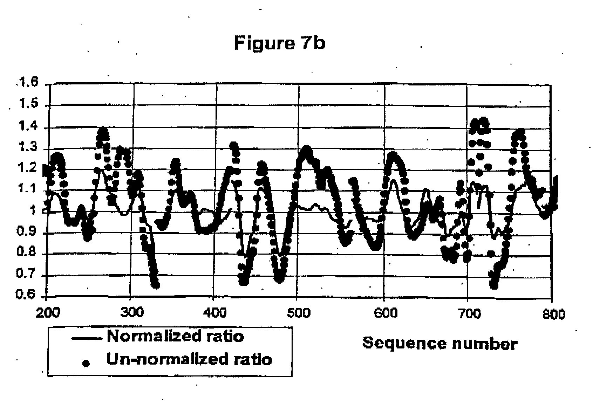

Figure 7b shows un-normalized and sequence-based normalized one-color ratios for a portion of the BRCA1 sequence. The normalization scheme is the same as in Figure 7a. The sequence-based normalization eliminates several false positives for this wild-type sample. Breaks in the normalized ratio curve occur at exon boundaries.

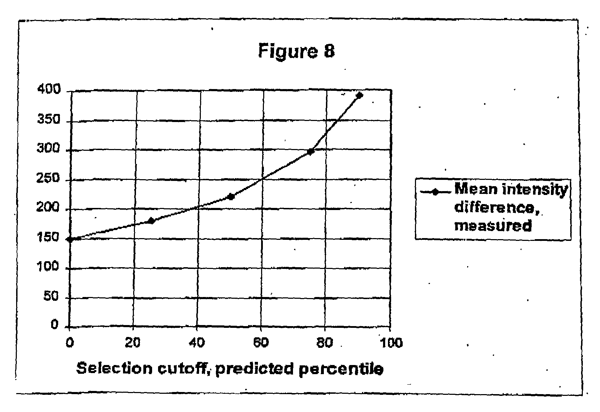

Figure 8 shows predicted intensity difference is used to select probe pairs with large intensity difference. Intensity differences are predicted using nearest-neighbor parameters derived from a BRCA1 data set. Probe pairs with predicted intensity differences above a cutoff value are selected. Here the cutoff is expressed as a percentile of all predicted intensity differences. A cutoff at the zero percentile corresponds to accepting all probe pairs, regardless of predicted intensity difference. A 50th percentile cutoff corresponds to selecting only those probe pairs with predicted intensity differences in the upper half of all predicted intensity differences. The measured intensity differences are from mRNA hybridized to a gene expression chip. By selecting probe pairs with large predicted intensity difference, one obtains probe pairs with larger actual intensity difference.

## DETAILED DESCRIPTION OF THE PREFERRED EMBODIMENTS

Reference will now be made in detail to the preferred embodiments of the invention. While the invention will be described in conjunction with the preferred embodiments, it will be understood that they are not intended to limit the invention to these embodiments. On the contrary, the invention is intended to cover alternatives, modifications and equivalents, which may be included within the spirit and scope of the invention.

As will be appreciated by one of skill in the art, the present invention may be embodied as a method, data processing system or program products. Accordingly, the present invention may take the form of data analysis systems, methods, analysis software, etc. Software written according to the present invention is to be stored in some form of computer readable medium, such as memory, or CD ROM, or transmitted over a network, and executed by a processor.

Computer software products may be written in any of various suitable programming languages, such as C, C++, Fortran and Java. The computer software product may be an independent application with data input and data display modules. Alternatively, the computer software products may be classes that may be instantiated as distributed objects. The computer software products may also be component software such as Java Beans, Enterprise Java Beans (EJB), Microsoft® COM/DCOM, etc.

Fig. 1 illustrates an example of a computer system that may be used to execute the software of an embodiment of the invention. Fig. 1 shows a computer system 1 that includes a display 3, screen 5, cabinet 7, keyboard 9, and mouse 11. Mouse 11 may have one or more buttons for interacting with a graphic user interface. Cabinet 7 houses a CD-ROM or DVD-ROM drive 13, system memory and a hard drive (see, Fig. 2) which may be utilized to store and retrieve software programs incorporating computer code that implements the invention, data for use with the invention and the like. Although a CD 15 is shown as an exemplary computer readable medium, other computer readable storage media including floppy disk, tape, flash memory, system memory, and hard drive may be utilized. Additionally, a data signal embodied in a carrier wave (e.g., in a network including the internet) may be the computer readable storage medium.

Fig. 2 shows a system block diagram of computer system 1 used to execute the software of an embodiment of the invention. As in Fig. 1, computer system 1 includes monitor 3, keyboard 9, and mouse 11. Computer system 1 further includes subsystems such as a central processor 51, system memory 53, fixed storage 55 (e.g., hard drive), removable storage 57 (e.g., CD-ROM), display adapter 59, sound card 61, speakers 63, and network interface 65. Other computer systems suitable for use with the invention may include additional or fewer subsystems. For example, another computer system may include more than one processor 51 or a cache memory. Computer systems suitable for use with the invention may also be embedded in a measurement instrument.

### I. Glossary

"Nucleic acids," according to the present invention, may include any polymer or oligomer of nucleosides or nucleotides, which include pyrimidine and purine bases, preferably cytosine, thymine, and uracil, and adenine and guanine, respectively. See Albert L. Lehninger, PRINCIPLES OF BIOCHEMISTRY, at 793-800 (Worth Pub. 1982). Indeed, the present invention contemplates any deoxyribonucleotide, ribonucleotide or peptide nucleic acid component, and any chemical variants thereof, such as methylated, hydroxymethylated or glucosylated forms of these bases, and the like. The polymers or oligomers may be heterogeneous or homogeneous in composition, and may be isolated from naturally-occurring sources or may be artificially or synthetically produced. See U.S. patent application Serial No. 08/630,427 which is incorporated herein by reference in its entirety for all purposes. In addition, the nucleic acids may be DNA or RNA, or a mixture thereof, and may exist permanently or transitionally in single-stranded or double-stranded form, including homoduplex, heteroduplex, and hybrid states. Oligonucleotide and polynucleotide are included in this definition and relate to two or more nucleic acids in a polynucleotide.

"Probe," as used herein, is defined as a nucleic acid, such as an oligonucleotide, capable of binding to a target nucleic acid of complementary sequence through one or more types of chemical bonds, usually through complementary base pairing, usually through hydrogen bond formation. As used herein, a probe may include natural (i.e. A, G, U, C, or T) or modified bases (7-deazaguanosine, inosine, etc.). In addition, the bases in probes may be joined by a linkage other than a phosphodiester bond, so long as it does not interfere with hybridization. Thus, probes may be peptide nucleic acids in which the constituent bases are joined by peptide bonds rather than phosphodiester linkages.

"Target nucleic acid" refers to a nucleic acid (often derived from a biological sample), to which the probe is designed to specifically hybridize. It is either the presence or absence of the target nucleic acid that is to be detected, or the amount of the target nucleic acid that is to be quantified. The target nucleic acid has a sequence that is complementary to the nucleic acid sequence of the corresponding probe directed to the target. The term target nucleic acid may refer to the specific subsequence of a larger nucleic acid to which the probe is directed or to the overall sequence (e.g., gene or mRNA) whose expression level it is desired to detect. The difference in usage will be apparent from context.

An "array" may comprise a solid support with peptide or nucleic acid probes attached to said support. Arrays typically comprise a plurality of different nucleic acid or peptide probes that are coupled to a surface of a substrate in different, known locations. These arrays, also described as "microarrays" or colloquially "chips" have been generally described in the art, for example, U.S. Pat. Nos. 5,143,854, 5,445,934, 5,744,305, 5,677,195, 6,040,193, 5,424,186 and Fodor et al., Science, 251:767-777 (1991). Each of which is incorporated by reference in its entirety for all purposes. These arrays may generally be produced using mechanical synthesis methods or light directed synthesis methods which incorporate a combination of photolithographic methods and solid phase synthesis methods. Techniques for the synthesis of these arrays using mechanical synthesis methods are described in, e.g., U.S. Pat. Nos. 5,384,261, and 6,040,193, which are incorporated herein by reference in their entirety for all purposes. Although a planar array surface is preferred, the array may be fabricated on a surface of virtually any shape or even a multiplicity of surfaces. Arrays may be peptides or nucleic acids on beads, gels, polymeric surfaces, fibers such as fiber optics, glass or any other appropriate substrate, see U.S. Patent Nos. 5,770,358, 5,789,162, 5,708,153, 6,040,193 and 5,800,992, which are hereby incorporated in their entirety for all purposes. Arrays may be packaged in such a manner as to allow for diagnostics or other manipulation of in an all inclusive device, see for example, US Patent Nos. 5,856,174 and 5,922,591, and 5,945,334, which are incorporated herein in their entirety by reference for all purposes. See also U.S. patent application Serial No. 09/545,207 which is incorporated herein in its entirety for all purposes for additional information concerning arrays, their manufacture, and their characteristics. It is hereby incorporated by reference in its entirety for all purposes.

## II. Models and Computational Methods

This invention is partially based upon a model of the sequence dependence of nucleic acid hybridization. One aspect of the invention provides methods, systems and software products for predicting the hybridization affinity between nucleic acid probes. The methods, systems and software products are particularly useful to enhance loss-of-signal footprints for detection of heterozygous mutations and to predict probe intensities for the design of gene expression chips. This work should also find wide application in other intensity normalization applications, chip manufacturing quality control, and basic understanding of the thermodynamics of hybridization on a chip.

Hybridization propensities may be described by energetic parameters derived from the probe sequence, and variations in hybridization and chip manufacturing conditions will result in changes in these parameters which can be detected and corrected.

In one aspect of the invention, models are developed to use energetic parameters to predict or fit nucleic acid hybridization affinity. Nucleic acid hybridization may involve a probe and a target nucleic acid. A probe generally refers to a nucleic acid that is used to determine qualitatively or quantitatively a target nucleic acid. A target nucleic acid is a nucleic acid of interest. A target molecule hybridizes to a probe with a favorable free energy, $\Delta G$, which is a function of the sequence. The hybridization affinity between the probe and the target molecules may be reflected by certain measurements. For example, if the probe is immobilized on a solid substrate, a target may be labeled with a marker and be contacted with the immobilized substrate to allow hybridization to occur. The hybridization affinity may be measured by determining the relative level of markers remaining after the substrate is washed. In preferred embodiments, the target molecules are labeled with a fluorescent marker. By determining the fluorescence intensity, the amount of remaining fluorescent marker after the wash, and thus the hybridization affinity, may be determined because the remaining intensity is proportional to the hybridization affinity.

Hybridization affinity may be given by Boltzmann's equation, exp ($\Delta G$ / RT), where T is the absolute temperature and R is the gas constant. Therefore the log of the intensity, I, is linearly related to $\Delta G$:

$$\log(I) = c + \Delta G \qquad \text{(equation 1)}$$

where c is a constant which is proportional to the log of the target concentration, and may be re-scaled to units of RT. (Throughout the specification, "log" refers to the natural log, with base e).

The dependence of $\Delta G$ on probe sequence can be quite complicated, but relatively simple models for $\Delta G$ have yielded good results. In general, $\Delta G$ is a function of the probe sequence of the form:

$$\Delta G(seq) = \Sigma_i \, P_i \, S_I \qquad \text{(equation 2)}$$

where the sum runs from 1 to the number of parameters; $P_i$ is the value of the ith parameter and $S_I$ is a corresponding value derived from the probe sequence, as explained below. A function which assigns a scalar value to points in a multi-dimensional space is called a functional of the space. The $S_I$'s are functionals of the probe sequence.

For example, by giving the values of S, one could use for a 5-mer probe. If one assigns a parameter to each probe sequence position and nucleotide type, for a probe with sequence GACTA, one might try the following values of S:

Table 1.

| S Values | | | | |
|---|---|---|---|---|
| I | position | Base | $S_i$ | |
| 1 | 1 | A | 0 | |
| 2 | 1 | C | 0 | |
| 3 | 1 | G | 1 | (1st position is G) |
| 4 | 1 | T | 0 | |
| 5 | 2 | A | 1 | (2nd position is A) |
| 6 | 2 | C | 0 | |
| 7 | 2 | G | 0 | |
| 8 | 2 | T | 0 | |
| 9 | 3 | A | 0 | |
| 10 | 3 | C | 1 | (3rd position is C) |
| 11 | 3 | G 0 | | | |
| 12 | 3 | T | 0 | |
| 13 | 4 | A | 0 | |
| 14 | 4 | C | 0 | |
| 15 | 4 | G | 0 | |
| 16 | 4 | T | 1 | (4th position is a T) |
| 17 | 5 | A | 1 | (5th position is an A) |
| 18 | 5 | C | 0 | |
| 19 | 5 | G | 0 | |
| 20 | 5 | T | 0 | |
| 21 | | | 1 | (one more value - this value is set to one for all sequences, and the corresponding parameter will be an over-all normalization |

The model and data permits the estimation of relative free energies. In mathematical terms, the above choice for S over-specifies the sequences and results in singular equations. Each probe position has one of four nucleotide types, so there are only three degrees of freedom per probe position. There are many possible ways around this problem. In some embodiments, the problem is solved by choosing one nucleotide type as a reference and using fewer parameters. If base T is chosen as a reference, S values are as follows:

Table 2.

| S Values When T is a Reference | | | | |
|---|---|---|---|---|
| I | position | Base | $S_i$ | |
| 1 | 1 | A | 0 | |
| 2 | 1 | C | 0 | |
| 3 | 1 | G | 1 | (1st position is G) |
| 4 | 2 | A | 1 | (2nd position is A) |
| 5 | 2 | C | 0 | |
| 6 | 2 | G | 0 | |
| 7 | 3 | A | 0 | |
| 8 | 3 | C | 1 | (3rd position is C) |
| 9 | 3 | G | 0 | |
| 10 | 4 | A | 0 | |
| 11 | 4 | C | 0 | |
| 12 | 4 | G | 0 | (4th position is T - which now has no corresponding parameter) |
| 13 | 5 | A | 1 | (5th position is A) |
| 14 | 5 | C | 0 | |
| 15 | 5 | G | 0 | |
| 16 | | | 1 | (for over-all normalization) |

In this representation, the Pi's will be the free energy of a base in a given position relative to a T in the same position.

With a functional form chosen for $\Delta G$ (seq) i.e., with a set of functionals $S_i$ chosen), one can determine the values of the parameters which give the best fit to the observed intensities in a set of data from a chip. The fitted intensity $I_{fit}$ is of the form

$$I_{fit} = \exp [\Sigma_i \, P_i \, S_I] \qquad \text{(equation 3)}$$

Where the sum is over all the parameters Pi and Si are the corresponding functionals of the probe sequence. The Pi are chosen to minimize the quantity

$$\Sigma_{probes} \{\log (Ifit) - \log (I_{msd})\}^2 = \Sigma_{probes} \{\Sigma_i P_i S_i - \log (I_{msd})\}^2 \qquad \text{(equation 4)}$$

where $I_{msd}$ is the measured intensity, the Pi are parameters to be determined and the $S_i$ are obtained from each probe sequence. This is a least-squares fit to the logs of the measured intensities. By choosing $I_{fit}$ as above, a linear least-square approach may be used to estimate the parameters.

The least-squares fit to determine the $P_i$ is computationally tractable for the tens of thousands of data points from a typical chip by direct solution of the normal equations using Cholesky decomposition *(see,* for example, Gene H. Golub and Charles F. van Loan: Matrix Computations, 2nd edn., The John Hopkins University Press, 1989, incorporated herein for all purposes). While some numerical methods texts warn against direct solution of the normal equations, this inventor has found them to be well-behaved even in single precision if the parameters are properly scaled. In preferred embodiments, double precision was used and no numerical problems were observed.

In some embodiments, the hybridization intensities, which is a measurement of hybridization affinity, are predicted using equation:

$$I = \exp\left[\sum_i P_i S_i\right].$$

The Pi values are obtained using, for example, a training data set. Once the Pi values are obtained, the hybridization intensities of a probe is calculated using the equation 1. One of skill in the art would appreciate that the Pi values may be dependent upon hybridization conditions, such as hybridization and subsequent wash temperature, ionic strength, etc. Therefore, a given set of Pi vales may only be applicable to a given hybridization condition.

**II. Refinements to the model**

One of skill in the art would appreciate that there are many possible variations on the hybridization model outlined above. The first is the use of polynomial fits to reduce the number of parameters required to describe the data. The second is the addition of parameters describing hairpin formation to the target molecule. The third refinement is the use of "nearest neighbor" parameters to improve the description of hybridization.

*A. Reduction of number of parameters*

Depending on the particular application and the size of the data, it may be desirable to reduce the number of parameters in the fit. One way of reducing the parameter is to replace the set of parameters for a given nucleotide type with a smooth function of probe base number. To return to the 5-mer example and the probe with sequence GACTA, one can choose to fit to second degree polynomials. The energetic parameters for each nucleotide type are taken to be quadratic functions of the base number. The values of S would be:

Table 3.

| S Values | | | | |
|---|---|---|---|---|
| I | Base | degree | $S_I$ | |
| 1 | A | 0 | 2 | (there are two A's) |
| 2 | A | 1 | 2 + 5 | (an A in position 2 and one in position 5) |
| 3 | A | 2 | 4 + 25 | (again, A at 2 and another at 5. This is $2^2 + 5^2$) |
| 4 | C | 0 | 1 | (one C) |
| 5 | C | 1 | 3 | (C is in position 3) |
| 6 | C | 2 | 9 | ($3_2$) |
| 7 | G | 0 | 1 | (one G) |
| 8 | G | 1 | 1 | (in position 1) |
| 9 | G | 2 | 1 | ($1^2$) |
| 10 | | | 1 | (for normalization) |

This representation reduces the number of parameters for 5-mer probes from 16 to 10.
For numerical reasons, one preferred implementation differs slightly from the one given above. The fit is made not to probe position but to the difference from the central position, and Legendre polynomials are used rather than a simple power series.
For 25-mer probes, in some preferred embodiments, 4th degree Legendre polynomials were used, resulting in 16 total parameters, as opposed to 76 without the polynomial fit.

*B. Probe-probe hybridization and hairpin information*

When data are fit with the above prescription, there are prominent regions where the fitted and measured intensities disagree. Many of these regions are due to probe-probe hybridization or hairpin formation in either the target or the probes. In one aspect of the invention, a probe-probe score and a hairpin score are used to describe the effects of these phenomena on hybridization intensity. The two scores differ in that sequences must have a loop of at least four bases to form a hairpin, whereas there is no such requirement for the bi-molecular probe-probe interaction.

*C. Nearest neighbor parameters*

Hybridization free energies depend not only on the identity of the bases involved, but also on their order. In particular, there are significant stacking effects between successive bases. These effects are called "nearest neighbor" interactions, and they can be made quantitative by assigning different hybridization free energies to different dinucleotide types. Thus DNA - RNA hybridization is usually described with 16 distinct free energies for AA, AC, AG, AT, CA, ..., TG, TT. DNA-DNA hybridization (in solution) requires only 8 parameters due to the symmetry between the two strands.

Applying a nearest neighbor description to fit hybridization data requires a change in the mathematics. If the above models are extended to include nearest-neighbor effects, and assign parameters to AA, AC, AG, AT, CA, ..., TC, TG (and leave TT as a reference) for positions 1-2, and then the same for all positions up to 24-25, the resulting normal equations are singular. This is because the parameters describe a space larger than that of true sequences. For example, it is not possible to simultaneously have AA in positions 1-2 and GG in positions 2-3, yet the parameters can describe such a situation.

One solution is to solve the normal equations with singular value decomposition rather than Cholesky decomposition. In effect, the computer constructs a basis set which excludes the null space which corresponds to impossible sequences, and finds the optimal solution in the remaining lower-dimensional space.

In one aspect of the invention, computer software products are provided to perform the hybridization intensity prediction. The software products contains code that performs the prediction using the equation:

$$I = \exp[\sum_i P_i S_i \ ].$$

The code may be stored in a suitable media and executed in a suitable digital computer. In some embodiments, the equation may be modified to account for probe-probe interaction, hairpin formation, nearest neighbor effect. The equation may also be modified to reduce the number of parameters as described above.

The software may include code that inputs Si, which are parameters related to the sequence of a probe. The assignment of S-values are illustrated in tables 1-3. The software may also include code for inputting Pi values, which are determined empirically as disclosed above. In addition, the software may include code for displaying resulting intensity value for a probe. In one exemplary software product, the software inputs the sequence of a probe, code are executed to assign S values based upon the sequence, Pi values are inputted from a relational database or a flat file. Intensity values are calculated and outputted to a display or for use of components of the software. For example, the intensity values may be used for probe selection (described below) in another module of the software product.

## III. Application to mutation detection

In one aspect of the invention, the model of hybridization developed above, methods and computer software for predicting nucleic acid hybridization are modified to correct for variations in hybridization or manufacturing conditions between two nucleic acid probe arrays. In some embodiments, the methods and computer software are used to improve detection of mutations, particularly heterozygous mutations. In some preferred embodiments, the methods and software may be used to improve the analysis of a loss-of-signal assay sensitive to all possible mutations (substitutions, insertions and deletions) of a given wild-type sequence. However, the methods and software may also be applied to detecting gain of signal if the mutant type is tiled for (for a more detailed description of tiling and mutation detection, *see, e.g., .* Methods for designing, selecting and making tiling probe sets are described in, for example, WO 95/11995, published on May 4, 1995, incorporated herein by reference for all purposes. Hacia *et al.* (1996, Detection of heterozygous mutations in BRCA1 using high density oligonucleotide arrays and two-color fluorescence analysis. Nat. Genet. 14: 441-447) describe a loss-of-signal assay which utilizes on- and off-chip references and two distinct fluorescent labels. In this "two-color" assay, the unknown target sample is labeled with one type of fluorescent label (e.g. red) while a wild-type reference is labeled with a second fluorescent label (e.g., green). On a second chip, wild-type target is labeled with both red and green and hybridized to the chip. For each perfect-match probe that is designed to be complementary to a target, a ratio of intensities is calculated:

$$\text{ratio} = [I(1, \text{wt, green}) I(2, \text{wt, red})]/[I(1,\text{unk,red}) I(2, \text{wt, green})] \qquad \text{(equation 4)}$$

where I(1, unk, red) is the intensity of the unknown target on chip 1, I(1, wt, green) is the intensity of the wild-type reference on the same chip, I(2, wt, red) is the intensity of the red-labeled wild-type sample on the reference chip, and I(2, wt, green) is the intensity of the green-labeled wild-type sample on the reference chip. The ratio is constructed so as to cancel out effects of chip-to-chip variation and effects due to differences between the two types of fluorescent labels. Hacia et al. then took a five-position moving window average of this ratio to signal the presence of a mutation. Values of the window-averaged ratio above a threshold were taken to indicate mutations.

The sequence-based normalization scheme involves fitting the ratio, either one-color or two-color, with the energetic parameters described above. Differences in the hybridization conditions are largely canceled out when the sequence-normalized ratio

$$\text{normalized ratio} = \text{ratio} *\exp [ -[\Sigma_i P_i S_I] \qquad \text{(equation 5)}$$

is formed, where Pi is the ith parameter of the fit to the ratio and Si is the corresponding value or functional of the probe sequence. Another way of expressing this is to say that the normalized ratio is the measured ratio divided by the fit to the ratio (i.e. a residual ratio). This normalization results in a large improvement of the performance of the one-color assay, and a more modest improvement in the performance of the two-color assay. Results of the one-color assay using sequence-based normalization yielded 6 out of 7 true positives in 7 samples, each consisting of 35 exons and 5.5 kb of sequence of the MSH2 and MLH1 genes. These results suggest that the method may be very useful in its present form as a preliminary screen, greatly limiting the amount of gel-based sequencing required. With a new chip design incorporating multiple copies of perfect match probes, further improvements in performance are possible.

The two-color assay performs very well without sequence-based normalization. The error rates seem low enough that it will require a fairly large amount of data (probably several hundred samples) to specify them, and also to specify what improvement sequence-based normalization will yield. Nevertheless, based on signal-to-noise ratios, sequence-based normalization seems likely to decrease the error rates of the two-color assay as well.

## IV. Application to probe selection for gene expression

The models developed here can yield predictions of probe intensities which are useful for selecting probes for gene expression.
In one implementation of the gene expression monitoring arrays (see, U.S. Patent Nos. 5,800,992 and 6,040,138, incorporated herein by reference for all purposes). Each gene is interrogated by a group of probe pairs. Each probe pair consists of a probe (perfect match) that is designed to be complementary to its target and a mismatch probe that is designed to contain a mismatch against its target. The intensity difference of each probe pair (perfect match minus mismatch) is predicted using the models described above. Figure 8 shows the effectiveness of this strategy. The data is from yeast mRNA hybridized to a gene expression chip. The intensity difference is predicted based on a nearest-neighbor fit to BRCA1 data. The plot shows the effects of using predicted intensity difference as a means of selecting probes for the design of gene expression chips. Probe pairs with large intensity difference are desired. A zero percentile cutoff means all probe pairs are included, while a 50th percentile cutoff means that only those probe pairs with predicted intensities in the upper half of the predictions are included. A factor of two increase in actual intensity difference is easily obtained by this method.

## V. Application to chip manufacturing quality control

In general the parameters and hybridization intensities will also reflect variations in the chip manufacturing process. A variation in the coupling efficiency of a synthesis step should have a multiplicative effect on probe intensity, like variations in hybridization free energy. For example, a synthesis step with 0.9 coupling efficiency followed by one with 0.85 efficiency results in 0.9 times 0.85 of the probes successfully completing both synthesis steps. As is the case with energetic terms, only relative efficiencies can be detected by the methods presented here, not absolute efficiencies.
For chips manufactured with the shift-mask process, energetic parameters corresponding to probe sequence number and base type also correspond to specific cycles in the manufacturing process. The parameters in figure 1b are an example of this case. If there were a synthesis step which was substantially less efficient than the others, one would expect a dip at the parameter corresponding to that step in figure 1b. For chips not made with shift-masks, parameters corresponding to specific cycles could also be constructed. Cycles with low yield should be detectable by relatively low values in the corresponding parameters derived from fitting the measured intensities.

**Examples**

Some embodiments of the methods of the invention were investigated using data from experiments examining mutation in the BRCA1 gene.

Germline mutations in BRCA1 are present in 50-60% of kindreds with breast and ovarian cancer, and may account for approximately 2-5% of all breast cancer cases in the general population (Hall et al., *Science* 250, 1684-1689 (1990), Narod et al., *Lancet* 338, 82-83 (1991), Easton et al. *Am. J. Hum. Genet.* 52, 678-701 (1993), Rowell et al., *Am. J. Hum. Genet.* 55, 861-865 (1994)). Heterozygous carriers are markedly predisposed to early onset breast and ovarian cancer, and are also at moderately increased risk of developing colon and prostate cancer (Ford et al., *Lancet* 343, 692-695 (1994)). The protein coding region of BRCA1 contains 5,592-bp in 22 coding exons spread over 100-kb of genomic DNA (Miki et al., *Science* 266, 66-71 (1994)). Over 111 unique BRCA1 mutations distributed throughout the gene have been described in the literature (Shattuck-Eidens et al., *Am. Med. Assoc.* 273, 535-541 (1995) and (Breast Cancer Information Core Database located on the *World Wide Web at http://www.nchgr.nih-gov/Intramural research/Lab-transfer/Bic/.))*. Most of these are frameshift, nonsense, or splice mutations resulting in a disruption of the normal reading frame. Except for the Ashkenazi Jewish population, where two mutations account for the majority of BRCA1 alterations (Struewing et al., *Am J. Hum. Genet.* 57, 1-7 (1995); Struewing et al., *Nature Genet. 11,* 198-200 (1995); Tonin et al., *Am. J. Hum. Genet.* 57, 189 (1995); Friedman et al., *Am. J. Hum. Genet.* 57, 1284-1297 (1995); FitzGerald et al., *N. Engl. J. Med.* 334, 143-149 (1996); Offit et al., *Lancet* 347, 1643-1645 (1996)), allelic heterogeneity confounds the ability to identify BRCA1 mutation carriers by methods (such as allele-specific oligonucleotide [ASO] hybridization) which detect only a finite set of previously described mutations.

A. Methods

(a) PCR from Genomic DNA and RNA Target Preparation

PCR reactions were performed on genomic samples using the EXPAND™ Long Range PCR Kit (Boehringer Mannheim) with primers 11FT3 5'-ATTAACCCTCACTAAAGGGAATTAAATGAAAGAGTATGAGC-3' and 11RT7 5'-TAATACGACTCACTATAGGGAGTGCTCCCAAAAGCATAAA-3' containing T3 and T7 RNA polymerase promoter sequences respectively. In vitro transcription reactions from these exon 11 amplicon templates were performed in 10 µl reaction volumes using T3 RNA polymerase transcription buffer (Promega), 0.7 MM of ATP, CTP, GTP, and UTP, 10 MM DTT, 0.7 MM fluorescein-12-UTP or 0.15 MM biotin-16-UTP (Boehringer Mannheim) for reference and test samples respectively, and 1OU T3 or T7 RNA polymerase as indicated.

(b) Target Preparation and Analysis

A reference template was generated from PCR amplification of exon 11 from a *BRCA1* cDNA clone. Reference and test sample transcription products were diluted to a final concentration of 100 nM in a 25 µl solution of 30 mm MgCl$_2$. The reaction was incubated at 94°C for 70 minutes to fragment targets (Lipshutz, et al., *BioTechniques* 19, 442-447 (1995); Kozal, et al., *Nature Med.* 2, 753-759 (1996)). Cofragmented targets were diluted 1/100 into a 300pl volume of hybridization buffer (3 M TMAC-Cl (tetramethylammonium chloride), 1x TE pH 7.4, 0.001% Triton X-100, 1 nM 5'-fluorescein-labelled control oligonucleotide 5'-CGGTAGCATCTTGAC-3'). This control oligonucleotide is designed to hybridize to specific surface probes to aid in image alignment. Target was hybridized with the chip in a 250 µl volume for 4 hours at 35°C. The chip surface was washed with 10 ml of wash buffer (6X SSPE, 0.001% Triton X-100) and stained with phycoerythrin-streptavidin conjugate (Molecular Probes) (2 µg/ml in wash buffer) for 5 minutes at room temperature. The chip was washed with 10 ml of wash buffer and scanned as described (Lipshutz et al., *BioTechniques* 19, 442-447 (1995); Kozal et al., *Nature Med.* 2, 753-759 (1996)). Hybridization signals were detected by a photomultiplier tube using 515-545 nm bandpass and 560 nm longpass emission filters for fluorescein reference (green) and biotin test (red) samples respectively (Cronin et al., *Hum. Mut.*7, 244-255 (1996); Chee et al., Science 274, 610-614 (1996)).

(d) Dideoxysequencing analysis

Four pairs of PCR primers (P1M13+ 5'-GTTTCCCAGTCACACGGAATTAAATGAAAG AGTATGAGC-3' and P1M13- 5'-AGGAAACAGCTATGACCATGTGAGGGGACGCTCT TG-3', P2M13+ 5'-GTTTCCCAGTCACACGTTGGGAAAACCTATCGGAA-3' and P2M135'-AGGAAACAGCTATGACCATCTTTGGGGTCTTCAGCA-3', P3M13+ 5'-GTTTCCCAG TCACACGTGTTCAAATACCAGTGAACTTA-3' and P3M13- 5'-AGGAAACAGCTATG ACCATGGAGCCCACT7CATTAGTAC-3', P4M13+ 5'-GTTTCCCAGTCACACGCCAAGT ACAGTGAGCACAATTA-3' and P4M13- 5'-AGGAAACAGCTATGACCATGTGCTCCC AAAAGCATAAA-3') were designed to generate four partially overlapping amplicons which cover the entire sequence of exon 11 and contain M13 forward and reverse sequences at the 5'-end of either strand Depending on region to be analyzed one of the four amplicons was generated from the appropriate genomic sample using the EXPAND™ Long Range PCR kit (Boehringer Mannheim) with the recommended protocol. Dye primer dideoxysequenciong reactions were performed using AmpliTaq DNA Polymerase FS kit (Perkin Elmer) with the suggested protocol and either M13 forward or M13 reverse DYEnamic™ energy transfer dye primers (Amersham Life Science).

(d) Data Analysis

Photomultiplier output signal was converted into proportional spatially addressed pixel values using Gene-Chip® Software (Affymetrix, Inc., Santa Clara, CA) to create a digitized fluorescence image. The relative contributions of the reference and test targets to each probe signal were extracted from each set of experimental green reference and red test images and imported into a Microsoft Excel 7.0a worksheet.

**B. Results**

(a) Prediction of Hybridization Affinity

Figure 3a shows a subset of experimental data from a BRCA1 chip and the fitted (predicted) intensities. Measured and fitted intensities (a measurement of hybridization affinity) for 400 bases (out of a total of 5.6 kb) of the BRCA1 sequence are shown. The fitted parameters are shown in Figure 3b. 97 parameters are used in the fit -- 75 base-type and position parameters (a parameter for each non-T base at each of the 25 probe positions) and one normalization parameter for each of the 22 exons. The separate normalization parameters for each exon allow for the fact that different exons maybe present at different concentrations. The root mean square (r.m.s.) deviation of the (natural) logs of $I_{fit}$ and $I_{msd}$ over the entire sequence is 0.8565, which indicates a good fit.

The parameters in Figure 3b have a simple interpretation. For example a C in the 10th position has a value of about 0.46. This means that the log of $I_{fit}$ for a probe with a C in the 10th position will be 0.46 greater than the log of $I_{fit}$ for a probe with a T in the 10th position and the same bases in all 24 other positions. In this data, where the target is RNA, one can see by examining Figure 3b that C's in the probe generally have the most favorable hybridization free energy, followed by G's, A's, and then T's.

The shapes of the curves in Figure 3b are in rough agreement with the shapes of footprints due to point mutations (or "smile plots"). As is seen with footprints, the curves peak near the middle of the probe sequence, indicating that positions near the middle have the largest effects on hybridization.

(b) Reduction of Parameters

Figure 4b shows the base- and position-dependent hybridization free energies derived from the fitting parameters used in Figure 4a. Each curve is a fourth degree polynomial specified by five of the fitting parameters. 37 parameters are used in the fit --15 specify the three fourth-degree polynomials describing the base-and position-dependence of the hybridization, and the remaining 22 are normalization parameters for each of the 22 exons. These curves are lower information content versions of those shown in Figure 3b. The r.m.s. deviation of the logs of $I_{fit}$ and $I_{msd}$ over the entire sequence is 0.8712 In spite of the large reduction in the number of parameters, the quality of the fit is degraded very little.

(c) Probe-Probe Hybridization and Hairpin Formation

Figures 5a and 5b are the same as Figures 4a and 4b, except that parameters corresponding to the probe-probe and hairpin scores were also included in the fit. 39 parameters are used in the fit -- 37 of them are as in Figure 4, and two additional ones describe probe-probe interactions and hairpin formation. The r.m.s. deviation of the logs of $I_{fit}$ and $I_{msd}$ over the entire sequence is 0.8274. One region where the fit improves is around sequence number 1980. Examination of the sequence around position 1980 shows that both probe-probe interactions and hairpins are likely there.

(d) Nearest Neighbor Parameters

Figures 6a-d show the results of using singular value decomposition approach using a 4th degree polynomial

fit to the nearest-neighbor free energies. 99 parameters are used in the fit -- 75 specify the 15 fourth-degree polynomials describing the base- and position-dependence of each non-reference nearest-neighbor term, two describe probe-probe interactions and hairpin formation, and 22 are normalization parameters for each of the 22 exons. The r.m.s. deviation of the logs of $I_{fit}$ and $I_{msd}$ over the entire sequence is 0.7436. Figures 6b-d show the base- and position-dependent nearest-neighbor hybridization free energies derived from the fitting parameters used in Figure 6a.

(e) Application in Mutation Detection.

Figure 7a shows the effect of sequence-based normalization on the one-color ratio for a portion of the sequence of BRCA1. The sequence-based normalization reduces the signal from potential false positives, while the signal from the true mutation at position 2798 is not diminished. Figure 7b shows the elimination of false positives by sequence-based normalization of a wild-type BRCA1 sample.

(d) Probe Selection for Gene Expression

Figure 8 shows the effectiveness of probe selection based upon predictions of probe intensities. The data is from yeast mRNA hybridized to a gene expression chip. The intensity difference of each probe pair (perfect match minus mismatch) is predicted based on a nearest-neighbor fit to BRCA1 data. The plot shows the effects of using predicted intensity difference as a means of selecting probes for the design of gene expression chips. Probe pairs with large intensity difference are desired. A zero percentile cutoff means all probe pairs are included, while a 50th percentile cutoff means that only those probe pairs with predicted intensities in the upper half of the predictions are included. A factor of two increase in actual intensity difference is easily obtained by this method.

**Conclusion**

The present inventions provide methods and computer software products for predicting nucleic acid hybridization affinity, detecting mutation, selecting probes, and improving probe array manufacturing quality control. It is to be understood that the above description is intended to be illustrative and not restrictive. Many variations of the invention will be apparent to those of skill in the art upon reviewing the above description. By way of example, the invention has been described primarily with reference to the use of a high density oligonucleotide array, but it will be readily recognized by those of skill in the art that the methods may be used to predict the hybridization affinity of other immobilized probes, such as probes that are immobilized in or on optical fibers or other supports. The basic methods and computer software of the invention may also be used to predict solution based hybridization. The scope of the invention should, therefore, be determined not with reference to the above description, but should instead be determined with reference to the appended claims, along with the full scope of equivalents to which such claims are entitled.

**Claims**

1. A computer implemented method for selecting probes for gene expression monitoring comprising:

   a) predicting hybridization intensities of a plurality of candidate probes and their corresponding control probes; and
   b) selecting the candidate probe that has the highest intensity difference over its corresponding control probe.

2. The method of claim 1 wherein said hybridization intensity (I) is determined using the equation:

$$I = \exp\left[\sum_i P_i S_i\right]$$

wherein said Pi is the value of the ith parameter and Si is a value derived from the sequence of said probe.

3. A computer implemented method for predicting hybridization affinity of a probe against its target comprising calculating hybridization intensity (I) using the equation:

$$I = \exp\left[\sum_i P_i S_i\right]$$

wherein said Pi is the value of the ith parameter and Si is a value derived from the sequence of said probe.

4. The method of claim 2 or claim 3 wherein said Pi is the free energy of a base in a given position of said sequence relative to a reference base selected from the group consisting of A, C, G and T.

5. The method of claim 3 wherein said Si is a functional of said sequence.

6. The method of any one of claims 2 to 3 wherein said Pi is determined empirically.

7. The method of claim 6 wherein said Pi is determined by least square fit using a training data set.

8. The method of claim 7 wherein Pi is determined using Cholesky decomposition.

9. The method of claim 8 wherein said Si for a given nucleotide type is replaced with a smooth function of probe base number.

10. The method of claim 8 wherein said Si for a given nucleotide type is replaced with a smooth function of the difference of a base to the central position.

11. The method of claim 9 or claim 10 wherein said smooth function is a polynomial function.

12. The method of claim 8 wherein said equation includes at least one parameter describing probe-probe hybridization.

13. The method of claim 8 wherein said equation includes at least one parameter describing hairpin formation.

14. The method of claim 8 wherein said equation includes parameters describing nearest neighbor interactions.

15. The method of claim 8 wherein said Pi is determined using singular value decomposition.

16. The method of any one of claims 1 to 15 wherein said probe is immobilized on a solid substrate.

17. A computer software product for predicting hybridization affinity of a probe against its target comprising:

computer program code for calculating hybridization intensity (I) using the equation:

$$I = \exp\left[\sum_i P_i S_i\right]$$

wherein said Pi is the value of the ith parameter and Si is a value derived from the sequence of said probe; and a computer readable media for storing said computer program code.

18. The computer software product of claim 17 wherein said Pi is the free energy of a base in a given position of said sequence relative to a reference base selected from the group consisting of A, C, G and T.

19. The computer software product of claim 18 wherein said Si is a functional of said sequence.

20. The computer software product of claim 19 wherein said Pi is determined empirically.

21. The computer software product of claim 20 wherein said Pi is determined by least square fit using a training data set.

22. The computer software product of claim 21 wherein Pi is determined using Cholesky decomposition.

23. The computer software product of claim 22 wherein said Si for a given nucleotide type is replaced with a smooth function of probe base number.

24. The computer software product of claim 22 wherein said Si for a given nucleotide type is replaced with a smooth function of the difference of a base to the central position.

25. The computer software product of claim 23 or claim 24 wherein said smooth function is a polynomial function.

26. The computer software product of claim 22 wherein said equation includes at least one parameter describing probe-probe hybridization.

27. The computer software product of claim 22 wherein said equation includes at least one parameter describing hairpin formation.

28. The computer software product of claim 22 wherein said equation includes parameters describing nearest neighbor interactions.

29. The computer software product of claim 22 wherein said Pi is determined using singular value decomposition.

30. A computer software product for selecting probes for gene expression monitoring comprising:

computer program code for predicting hybridization intensities of a plurality of candidate probes and their corresponding control probes;
computer program code for selecting the candidate probe that has the highest intensity difference over its corresponding control probe; and
a computer readable media for storing said codes.

31. The computer software product of claim 30 wherein said computer code for predicting hybridization intensity (I) comprising code for calculating using the equation:

$$\mathrm{I} = \exp\left[\sum_i P_i S_i\ \right]$$

wherein said Pi is the value of the ith parameter and Si is a value derived from the sequence of said probe.

32. The computer software product of claim 31 wherein said Pi is the free energy of a base in a given position of said sequence relative to a reference base selected from the group consisting of A, C, G and T.

33. The computer software product of claim 32 wherein said Si is a functional of said sequence.

34. The computer software product of claim 32 wherein said Pi is determined empirically.

35. The computer software product of claim 34 wherein said Pi is determined by least square fit using a training data set.

36. The computer software product of claim 35 wherein Pi is determined using Cholesky decomposition.

37. The computer software product of claim 36 wherein said Si for a given nucleotide type is replaced with a smooth function of probe base number.

38. The computer software product of claim 36 wherein said Si for a given nucleotide type is replaced with a smooth function of the difference of a base to the central position.

39. The computer software product of claim 36 or claim 38 wherein said smooth function is a polynomial function.

40. The computer software product of claim 36 wherein said equation includes at least one parameter describing probe-probe hybridization.

**41.** The computer software product of claim 36 wherein said equation includes at least one parameter describing hairpin formation.

**42.** The computer software product of claim 36 wherein said equation includes parameters describing nearest neighbor interactions.

**43.** The computer software product of claim 42 wherein said Pi is determined using singular value decomposition.

**44.** A computer program comprising program instructions for implementing on a computer a method as claimed in any of claims 1 to 16.

**45.** A computer program for predicting hybridization affinity of a probe against its target comprising program instructions for calculating on a computer hybridization intensity (I) using the equation:

$$I = \exp\left[\sum_i P_i S_i\right]$$

wherein said Pi is the value of the ith parameter and Si is a value derived from the sequence of said probe.

**46.** A computer program for selecting probes for gene expression monitoring comprising program instructions for implementing on a computer the steps of predicting hybridization intensities of a plurality of candidate probes and their corresponding control probes;
    selecting the candidate probe that has the highest intensity difference over its corresponding control probe.

**47.** A computer configured to implement a program as claimed in any of claims 44 to 46.

**48.** A computer readable medium storing instructions for implementing a program as claimed in any of claims 44 to 46.

**Figure 1**

Figure 2

## Figure 3a

Figure 3b

Figure 4a

Figure 4b

## Figure 5a

Figure 5b

Figure 6a

Figure 6b

Figure 6c

Figure 6d

Figure 7a

Figure 7b

Figure 8